# FASCICULE DE BREVET EUROPEEN

(11) **EP 1 113 014 B1**
(45) Date de publication et mention de la délivrance du brevet: **27.04.2005**
(21) Numéro de dépôt: 00403673.7
(22) Date de dépôt: 26.12.2000
(51) Int. Cl.: C07D 401/12, A61K 31/454, A61P 9/00, C07D 211/06, C07D 209/04, C07D 401/06, C07D 405/14, A61K 31/445, A61K 31/415, A61K 31/505, C07D 417/06

(54) **Urées linéaires ou cycliques, leur procédé de préparation et les compositions pharmaceutiques qui les contiennent**
Lineare oder zyklische Harnstoffe, Verfahren zu ihrer Herstellung und die enthaltende pharmazeutische Zusammensetzungen
Linear or cyclic ureas, a process for their preparation and pharmaceutical compositions containing them

(30) Priorité: 30.12.1999 FR 9916701
(43) Date de publication de la demande: 04.07.2001
(60) Demande divisionnaire: 02078578.8
(73) Titulaire: LES LABORATOIRES SERVIER, 92200 Neuilly sur Seine (FR)
(72) Inventeur: Peglion, Jean-Louis, 78110 Le Vesinet (FR); Poitevin, Christophe, 75019 Paris (FR); Vilaine, Jean-Paul, 92290 Chatenay-Malabry (FR); Villeneuve, Nicole, 92500 Rueil-Malmaison (FR); Bourguignon, Marie-Pierre, 78400 Chatou (FR); Thollon, Catherine, 75011 Paris (FR)

(56) Documents cités:
- EP-A- 0 597 112
- EP-A- 0 811 622
- EP-A- 0 870 763
- WO-A-92/00282
- WO-A-94/13659
- WO-A-95/33729
- WO-A-97/28157
- WO-A-98/02435
- FR-A- 2 196 809
- FR-A- 2 303 541
- FR-A- 2 338 940
- FR-A- 2 761 069
- BATEY R A ET AL: "An Efficient New Protocol for the Formation of Unsymmetrical Tri- and Tetrasubstituted Ureas" TETRAHEDRON LETTERS,ELSEVIER SCIENCE PUBLISHERS, AMSTERDAM,NL, vol. 39, no. 35, 27 August 1998 (1998-08-27), pages 6267-6270, XP004128844 ISSN: 0040-4039
- DILLON G.A. ET AL: 'Nitric oxide and endothelial dysfunction' CONTEMPORARY CARDIOLOGY vol. 4, no. 13, pages 207 - 225
- EBERHARDT R.T. ET AL: 'Nitric oxide in atherosclerosis' CONTEMPORARY CARDIOLOGY vol. 4, no. 16, pages 273 - 295
- SCHäCHINGER V. ET AL: 'Prognostic implications...' CORONARY ARTERY DISEASE vol. 12, 2001, pages 435 - 443
- NAPOLI C. ET AL: 'Nitric oxide and atherosclerosis' NITRIC OXIDE: BIOLOGY AND CHEMISTRY vol. 5, no. 2, 2001, pages 88 - 97
- CUNNINGHAM OWENS D.G.: 'adverse effects...' DRUGS vol. 51, no. 6, June 1996, pages 895 - 930

## Description

La présente invention concerne de nouvelles urées linéaires ou cycliques, leur procédé de préparation et les compositions pharmaceutiques qui les contiennent.

Les composés de la présente invention sont utiles dans le traitement des maladies ou des conditions pathologiques dans lesquelles une dysfonction endothéliale est connue comme étant un mécanisme pathogène et/ou aggravant. Ces pathologies sont : l'athérosclérose, l'existence de facteurs de risque vasculaire (dyslipidémie, diabète, hypertension artérielle systémique), les différentes formes cliniques d'ischémie myocardique ou périphérique, l'insuffisance cardiaque et les différentes formes d'hypertension artérielle pulmonaire. Ces composés sont également utiles pour le traitement de patients qui subissent une transplantation cardiaque ou une reperméabilisation vasculaire telle qu'un pontage, une thrombolyse ou une dilatation artérielle avec ou sans stent.

Une diminution de la disponibilité vasculaire en monoxyde d'azote (NO) représente le mécanisme majeur de la dysfonction endothéliale observée dans les maladies et conditions pathologiques précédemment citées et explique son rôle pathogène (*Cardiovasc. Res*., **1999,** 43, 572 ; *Coronary. Art. Dis*. **1999,** 10, 277 ; *Coronary. Art. Dis*., **1999,** 10, 301 ; *Coronary. Art. Dis*., **1999,** 10, 287 ; *Coronary. Art. Dis*., **1999,** 10, 295).

En effet, dans ces conditions pathologiques, la dysfonction endothéliale peut résulter de deux mécanismes principaux: 1) une insuffisance de production de NO liée à l'inhibition de la NO synthase endothéliale par des inhibiteurs endogènes tel l'ADMA (asymétrique diméthyle arginine) dont la concentration plasmatique augmente chez des patients présentant des facteurs de risque cardiovasculaire (*Cardiovasc. Res.,* **1999,** 43, 542 ; *Hypertension*, **1997,** 29, 242 ; *Circulation*, **1997,** 95, 2068), 2) une inactivation du NO par l'anion superoxyde (O₂⁻) dont la production est accrue dans des conditions pathologiques (*Cardiovasc. Res.,* **1999,** 43, 562 ; *Eur.J Biochem.* **1997,** 245, 541 ; *J. Clin. Invest.,* **1993,** 91 2546).

Le NO exerce dans les conditions normales des effets majeurs tels que: 1) la régulation de la vasomotricité artérielle par son effet vasodilatateur (*N Engl. J Med*., **1993,** 329, 2002 ; *Nature,* **1980,** 288, 373), 2) la limitation de l'adhésion et de l'agrégation plaquettaire (*Trends Pharmacol. Sci*., **1991,** 12, 87), 3) le contrôle de l'adhésion aux cellules endothéliales de leucocytes et de monocytes (*Proc. Natl Acad. Sci. USA,* **1991,** 88, 4651), 4) l'inhibition de la prolifération des cellules musculaires lisses vasculaires (*Cardiovasc*. *Res*., **1999,** 43, 580, *Circulation*, **1993,** 87 V51)
Ceci explique que la déficience en NO au niveau de la paroi artérielle favorise des phénomènes pathologiques comme la vasoconstriction, la thrombose, l'accumulation lipidique et la prolifération des cellules musculaires lisses vasculaires.

Des expériences *in vitro* ont permis de démontrer que les composés de la présente invention permettent de limiter la dysfonction endothéliale et la diminution de disponibilité vasculaire en NO qui ont été induites par des tests impliquant les deux mécanismes physiopathologiques déjà cités : l'inhibition de la NO synthase endothéliale et un stress oxydatif par production d'O₂⁻.

Ainsi, grâce à leur activité pharmacologique spécifique, capable de limiter le développement de la dysfonction endothéliale, les composés de la présente invention, outre le fait qu'ils soient nouveaux, sont utiles pour prévenir le développement, l'extension et les complications des lésions athéroscléreuses notamment chez les patients présentant un facteur de risque vasculaire (dyslipidémie, diabète, hypertension artérielle), pour traiter les différentes formes cliniques d'ischémie myocardique ou périphérique, l'insuffisance cardiaque, les différentes formes d'hypertension artérielle pulmonaire. Ces composés sont également utilisés pour prévenir les complications vasculaires (spasme, thrombose, resténose, athérosclérose accélérée) chez les patients qui subissent un pontage, une dilatation vasculaire avec ou sans stent ou d'autres formes de reperméabilisation vasculaire ainsi qu'une transplantation cardiaque.

Des composés de structure voisine ont été décrits dans la littérature. C'est le cas plus particulièrement des demandes de brevets FR 2 303 541, FR 2 196 809, EP 811 622, WO 94/13659, WO 97/28157 et WO 92/00282, qui revendiquent des composés présentant notamment une urée cyclique, lesdits composés étant utiles dans le traitement des maladies du système nerveux central telles que la dépression ou la psychose et /ou les maladies cardiovasculaires. De même, le brevet FR 2 338 940 décrit des dérivés présentant notamment un motif 1-{1-[2-hydroxy-3-(aryloxy)-propyl]-4-pipéridyl}-3-aryl-imidazolidine-2-one, et les revendique pour leur utilité dans le traitement de l'hypertension vasculaire. Enfin, le brevet EP 0 526 342 décrit de nouvelles (isoquinoléin-5-yl)sulfonamides, lesquelles sont utiles dans le traitement de l'ischémie myocardique.

Les composés de la présente invention se distinguent nettement de cet art antérieur, tant dans leur structure chimique même, que dans leur activité pharmacologique spécifique de la protection endothéliale.

Les composés de l'invention sont les composés de formule (IA) : 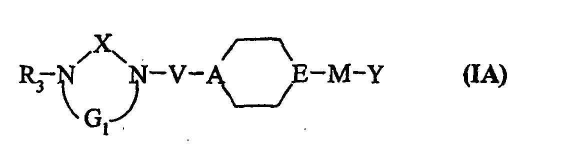 dans laquelle :
- R₃: représente un groupement aryle, et avantageusement un groupement phényle, éventuellement substitué par un groupement choisi parmi atome d'halogène, groupement hydroxy, alkoxy (C₁-C₆) linéaire ou ramifié, arylalkoxy (C₁-C₆) linéaire ou ramifié, nitro, trihalogénoalkyle (C₁-C₆) linéaire ou ramifié, et cyano,
- X: représente un groupement carbonyle,
- G₁: représente une chaîne alkylène (C₂-C₃) linéaire,
- V: représente une chaîne alkylène (C₁-C₆) linéaire ou ramifiée,
- A: représente un atome d'azote quand E représente un groupement CH, ou
- A: représente un groupement CH quand E représente un atome d'azote,
- M: représente une chaîne alkylène (C₁-C₄) linéaire ou ramifiée,
- Y: représente un groupement benzofuran-3-yl ou un groupement phényloxy éventuellement substitué par un groupement choisi parmi atome d'halogène, groupement alkylsulfonyle (C₁-C₆) linéaire ou ramifié, et alkylthio (C₁-C₆) linéaire ou ramifié,
leurs isomères, leurs hydrates, leurs solvates, ainsi que leurs sels d'addition à un acide pharmaceutiquement acceptable,
étant entendu que par groupement aryle, on comprend un groupement choisi parmi phényle, biphényle, naphtyle, dihydronaphtyle, tétrahydronaphtyle, indanyle, indényle, et benzocyclobutyle.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique.

D'une façon préférentielle, les composés préférés de l'invention sont les composés de formule (IA) telle que définie précédemment dans laquelle :
* A représente un atome d'azote et E représente un groupement CH quand Y représente un groupement phényloxy éventuellement substitué,
* ou A représente un groupement CH et E représente un atome d'azote quand Y représente un groupement benzofuran-3-yl.

D'une autre façon préférentielle, les composés préférés de l'invention sont les composés de formule (IA) telle que définie précédemment dans laquelle Y représente un groupement phényloxy éventuellement substitué.

Le composé préféré de l'invention est la 1-(2-{4-[2-(4-fluorophénoxy)éthyl]-1-pipéridinyl}éthyl)-3-phényl-2-imidazolidinone.

Les isomères ainsi que les hydrates, les solvates et les sels d'addition à un acide pharmaceutiquement acceptable des composés préférés font partie intégrante de l'invention.

L'invention s'étend également au procédé de préparation des composés de formule (I) caractérisé en ce qu'on utilise comme produit de départ un composé de formule (II) :

R₃ - N = C = O **(II)**

dans laquelle R₃ est tel que défini dans la formule (I),
que l'on fait réagir :
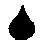 soit avec un composé de formule (III) :

   HO - G₁ₐ - NH - Vₐ - OH **(III)**

   dans laquelle Vₐ représente une chaîne alkylène (C₂-C₆) et G₁ₐ représente une chaîne alkylène linéaire en (C₂-C₃),
   pour conduire aux composés de formule (IVa) : 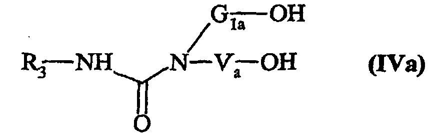 dans laquelle R₃, G₁ₐ, et Vₐ sont tels que définis précédemment,
   composés de formule (IVa) qui sont transformés par les méthodes classiques de la synthèse organique, pour conduire aux composés de formule (IVb) : 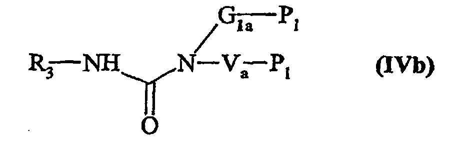 dans laquelle P₁ représente un atome de chlore, de brome, d'iode, ou un groupement OSO₂R₄ dans lequel R₄ représente un groupement méthyle, trifluorométhyle ou tolyle, R₃, Vₐ et G₁ₐ sont tels que définis précédemment,
   composés de formule (IVb) que l'on soumet à l'action de la chaleur,
   pour conduire aux composés de formule (IVc) : 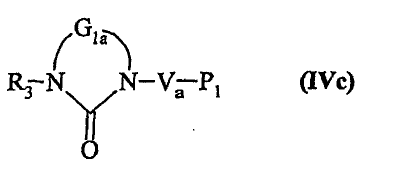 dans laquelle R₃, G₁ₐ, Vₐ et P₁ sont tels que définis précédemment, composés de formule (IVc) qui sont traités en condition basique par un composé de formule (V) : 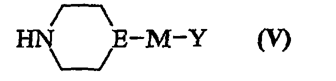 dans laquelle E, M et Y sont tels que définis dans la formule (I),
   pour conduire aux composés de formule (I/a), cas particulier des composés de formule (I) : 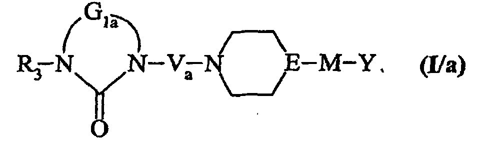 dans laquelle R₃, G₁ₐ, Vₐ, E, M et Y sont tels que définis précédemment,
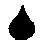 soit avec un composé de formule (IX) : 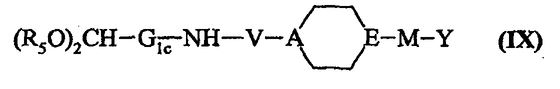 dans laquelle G_{1c} représente une chaîne alkylène linéaire en C₁ ou C₂, R₅ représente un groupement alkyle (C₁-C₆) linéaire ou ramifié, V, A, E, M et Y sont tels que définis dans la formule (I),
   pour conduire aux composés de formule (X) : 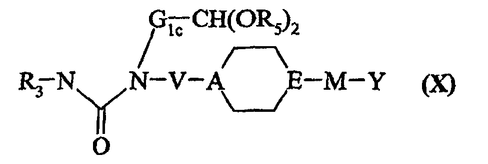 dans laquelle R₃, G_{1c}, V, A, R₅, M et Y sont tels que définis précédemment, composés de formule (X) que l'on place en présence d'acide organique fort,
   pour conduire aux composés de formule (I/c) : 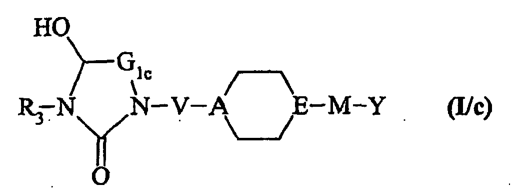 dans laquelle R₃, G_{1c}, V, A, E, M et Y sont tels que définis précédemment, composé de formule (I/c) que l'on hydrogène selon des techniques classiques de la synthèse organique, pour conduire aux composés de formule (I/d), cas particulier des composés de formule (I) : 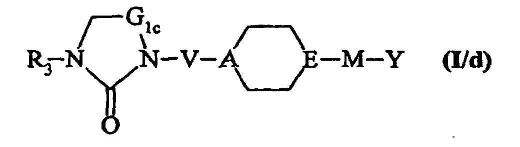 dans laquelle R₃, G_{1c}, V, A, E, M et Y sont tels que définis précédemment,
   l'ensemble des composés de formule (I/a) et (I/d) forment les composés de l'invention, que l'on purifie, le cas échéant, selon des techniques classiques de purification, dont on sépare éventuellement les isomères selon une technique classique de séparation, et que l'on transforme, si on le souhaite, en ses hydrates, ses solvates ou ses sels d'addition à un acide pharmaceutiquement acceptable.

Les composés de formule (II), (III), (V) et (IX) sont soit des composés commerciaux, soit obtenus selon des méthodes connues et classiques de la synthèse organique.

Les composés de la présente invention sont utiles dans le traitement des maladies ou des conditions pathologiques dans lesquelles une dysfonction endothéliale est connue. De ce fait, grâce à leur activité pharmacologique spécifique, les composés de l'invention sont utiles pour prévenir le développement, l'extension et les complications des lésions athéroscléreuses, en particulier chez les patients présentant un facteur de risque vasculaire (dyslipidémie, diabète, hypertension artérielle systémique), dans le traitement de l'ischémie myocardique ou périphérique, de l'insuffisance cardiaque, de l'hypertension artérielle pulmonaire, pour la prévention des complications vasculaires après pontage vasculaire, dilatation vasculaire, reperméabilisation vasculaire et transplantation cardiaque.

La présente invention a également pour objet les compositions pharmaceutiques contenant comme principe actif au moins un composé de formule (I), ses isomères optiques, ses hydrates, ses solvates, ses sels d'addition à un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 1 mg à 200 mg en une ou plusieurs prises par jour.

Les exemples suivants illustrent l'invention mais ne la limitent en aucune façon. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse, ...).

Les points de fusion ont été déterminés soit à la platine chauffante de Kofler (K.), soit à la platine chauffante sous microscope (M.K.).

### PREPARATION 1 : 4-(p-fluorophénoxyméthyl)pipéridine

### Stade 1 : N-t-butyloxycarbonyl-4-(p-fluorophénoxyméthyl)pipéridine

Sur un mélange de 13,5 g de N-*t*-butyloxycarbonyl-4-hydroxyméthyl pipéridine, 7,45 g de *p*-fluorophénol, 17,4 g de triphénylphosphine et 140 ml de tétrahydrofurane sont versés, en 30 minutes, 10,5 ml d'azodicarboxylate de diéthyle. Après 12 heures d'agitation à température ambiante, le milieu réactionnel est concentré, repris à l'éther, lavé à l'eau puis à la soude 1*N*, puis lavé à l'eau. Après séchage, filtration et évaporation sous pression réduite, une chromatographie sur gel de silice (cyclohexane/acétate d'éthyle : 90/10) permet d'isoler le produit attendu.
***Point de fusion*** ***(K) : 94-98°C***

### Stade 2 : 4-(p-fluorophénoxyméthyl)pipéridine

12,1 g du produit obtenu au stade 1 est traité à température ambiante par 300 ml d'une solution d'éther chlorhydrique pendant 48 heures permettant d'obtenir le produit titre sous forme de chlorhydrate. La base libre est obtenue par traitement du chlorhydrate par de la soude 1*N* suivi d'une extraction au dichlorométhane.

### PREPARATION 2 : 4-[2-(p-fluorophénoxy)éthyl]pipéridine

Le produit est obtenu selon le procédé de la préparation 1 en utilisant au stade 1 le 2-(*N-t*-butyloxycarbonyl-4-pipéridinyl)éthanol comme substrat.

### PREPARATION 3 : 4-(phénoxyméthyl)pipéridine

Le produit est obtenu selon le procédé de la préparation 1 en utilisant au stade 1 le phénol à la place du *p*-fluorophénol.

### PREPARATION 4 : 4-(p-méthylsulfonylphénoxyméthyl)pipéridine

Le produit est obtenu selon le procédé de la préparation 1 en utilisant au stade 1 le *p*-méthylsulfonylphénol à la place du *p*-fluorophénol.
***Point de fusion*** ***(chlorhydrate) : 230-234°C***

### PREPARATION 5 : 4-[2-(p-méthylsulfonylphénoxy)éthyl]pipéridine

Le produit est obtenu selon le procédé de la préparation 1 en utilisant au stade 1 le 2-(*N-t*-butyloxycarbonyl-4-pipéridinyl)éthanol et le *p*-méthylsulfonylphénol.

### PREPARATION 17 : 2-{4-[2-(p-fluorophénoxy)éthyl]-1-pipéridinyl}éthylamine

### Stade 1 : {4-[2-(p-fluorophénoxy)éthyl]-1-pipéridinyl}acétonitrile

Dans 320 ml de méthylisobutylcétone sont ajoutés 92,4 mmol du produit de la préparation 2, 6,5 ml de bromoacétonitrile, et 39,2 g de carbonate de potassium. Après 12 heures à reflux, le milieu réactionnel est filtré, concentré sous pression réduite. Le résidu est repris à l'eau et au dichlorométhane. La phase organique est lavée à l'eau, séchée, filtrée puis concentrée sous pression réduite permettant d'obtenir le produit attendu.
***Point de fusion*** ***(K) : 72°C***

### Stade 2 : 2-{4-[2-(p-fluorophénoxy)éthyl]-1-pipéridinyl}éthylamine

A une suspension de 81 mmol de LiAlH₄ dans 200 ml de tétrahydrofurane, maintenue à 0°C, est additionnée une solution du produit obtenu au stade 1 dissout dans 200 ml de tétrahydrofurane. Après 12 heures d'agitation à température ambiante, le milieu réactionnel est hydrolysé par 2,75 ml d'eau, 2,2 ml de soude à 20 %, puis 10 ml d'eau. Après filtration et rinçage par du tétrahydrofurane, le filtrat est séché puis concentré sous pression réduite permettant d'isoler le produit attendu.

### PREPARATION 23 : 1-(2-chloroéthyl)-3-phényl-2-imidazolidinone

### Stade 1 : 1,1-bis-(2-hydroxyéthyl)-3-phényl urée

A une solution de 0,168 mol d'isocyanate de phényle dans 40 ml de dichlorométhane est additionnée, à 10°C, 0,163 mol de diéthanolamine dissoute dans 160 ml de dichlorométhane. Après 1 heure de réaction à 10°C, puis 12 heures à température ambiante, le milieu réactionnel est concentré sous pression réduite.

### Stade 2 : 1,1-bis-(2-chloroéthyl)-3-phényl urée

A une solution de 0,173 mol du produit obtenu dans le stade 1 dans 100 ml de dichlorométhane sont additionnés, à 0°C, 26,44 ml de chlorure de thionyle. Après 4 heures de réaction à reflux, puis 12 heures à température ambiante, le milieu réactionnel est concentré sous pression réduite.

### Stade 3 : 1-(2-chloroéthyl)-3-phényl-2-imidazolidinone

0,167 mol du produit obtenu au stade 2 est chauffée 3 heures à 120°C puis 6 heures à 140°C. A la fin du dégagement gazeux, on refroidit. Une chromatographie sur gel de silice (dichlorométhane) permet d'isoler le produit attendu.
***Point de fusion*** ***(K) : 92°C***

### PREPARATION 24 : 1-(2-chloroéthyl)-3-(p-méthoxyphényl)-2-imidazolidinone

Le produit est obtenu selon le procédé de la préparation 23 en utilisant au stade 1 l'isocyanate de *p*-méthoxyphényle.
***Point de fusion*** ***(K) : 118°C***

### PREPARATION 25 : 1-(2-chloroéthyl)-3-(p-hydroxyéthyl)-2-imidazolidinone

Le produit est obtenu lors de la purification sur gel de silice de la préparation 24 et est isolé dans un rapport 1/2.

### Point de fusion (K) : 171°C

### PREPARATION 26 : 1-(2-chloroéthyl)-3-(o-chlorophényl)-2-imidazolidinone

Le produit est obtenu selon le procédé de la préparation 23 en utilisant au stade 1 l'isocyanate de *o*-chlorophényle.

### PREPARATION 27 : 1-(2-chloroéthyl)-3-(p-fluorophényl)-2-imidazolidinone

Le produit est obtenu selon le procédé de la préparation 23 en utilisant au stade 1 l'isocyanate de *p*-fluorophényle.
***Point de fusion*** ***(K) : 105°C***

### PREPARATION 28 : 3-(p-benzyloxyphényl)-1-(2-chloroéthyl)-2-imidazolidinone

Le produit est obtenu selon le procédé de la préparation 23 en utilisant au stade 1 l'isocyanate de *p*-benzyloxyphényle.
***Point de fusion*** ***(M.K.) : 128-133°C***

### PREPARATION 29 : 1-(2-chloroéthyl)-3-(p-nitrophényl)-2-imidazolidinone

Le produit est obtenu selon le procédé de la préparation 23 en utilisant au stade 1 l'isocyanate de *p*-nitrophényle.
***Point de fusion*** ***(M.K.) : 134°C***

### PREPARATION 30 : 1-(2-chloroéthyl)-3-(p-chlorophényl)-2-imidazolidinone

Le produit est obtenu selon le procédé de la préparation 23 en utilisant au stade 1 l'isocyanate de p-chlorophényle.
***Point de fusion*** ***(M.K.) : 112-114°C***

### PREPARATION 31 : 4-[2-(p-Chlorophénoxy)éthyl]pipéridine

Le produit est obtenu selon le procédé de la préparation 1, des stades 1 à 2, en utilisant comme substrats le 2-(N-*t*-butyloxycarbonyl-4-pipéridinyl)éthanol et le p-chlorophénol.
***Point de fusion du chlorhydrate*** ***(K.) : 205°C***

### PREPARATION 32 : 4-[(p-Méthylthiophénoxy)méthyl]pipéridine

Le produit est obtenu selon le procédé de la préparation 1, des stades 1 à 2, en utilisant comme substrat le p-méthylthiophénol.
***Point de fusion du chlorhydrate*** ***(M.K.) : 206-210°C***

### PREPARATION 33 : 4-[2-(p-Méthylthiophénoxy)éthyl]pipéridine

Le produit est obtenu selon le procédé de la préparation 1, des stades 1 à 2, en utilisant comme substrats celui de la préparation 2 et celui de la préparation 32.
***Point de fusion du chlorhydrate*** ***(M.K.) : 146-150°C***

### PREPARATION 34 : 4-[2-(Phénoxy)éthyl]pipéridine

Le produit est obtenu selon le procédé de la préparation 1, des stades 1 à 2, en utilisant comme substrats le 2-(N-*t*-butyloxycarbonyl-4-pipéridinyl)éthanol et le phénol.
***Point de fusion du chlorhydrate*** ***(K.) : 155°C***

### PREPARATION 35 : 1-(2-Chloroéthyl)-3-(p-trifluorométhylphényl)-2-imidazolidinone

Le produit est obtenu selon le procédé de la préparation 23, des stades 1 à 3, en utilisant comme substrat au stade 1, l'isocyanate de p-trifluorométhylphényle.
***Point de fusion*** ***(K.) : 70°C***

### PREPARATION 36 : 1-(2-Chloroéthyl)-3-(p-cyanophényl)-2-imidazolidinone

Le produit est obtenu selon le procédé de la préparation 23, des stades 1 à 3, en utilisant comme substrat au stade 1 l'isocyanate de p-cyanophényle.
***Point de fusion*** ***(K.) : 149°C***

### PREPARATION 37 : N-(2,2-Diméthoxyéthyl)-N-(2-{4-[2-(4-fluorophénoxy)éthyl]-1-pipéridinyl}éthyl)amine

Le produit est obtenu sous forme d'une huile selon le procédé de la préparation 9 en utilisant comme substrat le produit de la préparation 17.

### EXEMPLE 5 : 1-(2-{4-[2-(p-Fluorophénoxy)éthyl]-1-pipéridinyl}éthyl)-3-phényl-2-imidazolidinone et son chlorhydrate

7,72 mmol du produit de la préparation 2 et 7,72 mmol du produit de la préparation 23 sont dissous dans 70 ml de méthylisobutylcétone, puis 3,19 g de carbonate de potassium sont ajoutés. Après 12 heures de réaction au reflux, le milieu est filtré puis évaporé. Le résidu est repris à l'acétate d'éthyle, puis lavé à l'acétate d'éthyle, séché, filtré et concentré. Une chromatographie sur gel de silice (dichlorométhane/méthanol/hydroxyde d'ammonium : 95/5/0,5) permet d'isoler le produit attendu qui est transformé en son chlorhydrate par une solution d'éthanol chlorhydrique.
***Point de fusion*** ***(M.K.) : 176-180°C***

| ***Microanalyse élémentaire*** **:** | | | | |
|---|---|---|---|---|
| | ***C*** | ***H*** | ***N*** | ***Cl*** |
| ***% trouvé*** | ***64,27*** | ***7,05*** | ***9,27*** | ***8,28*** |
| ***% calculé*** | ***64,35*** | ***6,97*** | ***9,38*** | ***7,91*** |

### EXEMPLE 6 : 1-(2-{4-[2-(p-Fluorophénoxy)éthyl]-1-pipéridinyl}éthyl)-3-(p-méthoxyphényl)-2-imidazolidinone et son chlorhydrate

Le produit est obtenu selon le procédé de l'exemple 5 en utilisant le produit de la préparation 24 à la place de celui de la préparation 23.
***Point de fusion*** ***(M.K.) : 197-203°C***

| ***Microanalyse élémentaire*** **:** | | | | |
|---|---|---|---|---|
| | ***C*** | ***H*** | ***N*** | ***Cl*** |
| ***% trouvé*** | ***62,78*** | ***7,17*** | ***8,82*** | ***7,82*** |
| ***% calculé*** | ***62,82*** | ***6,97*** | ***8,79*** | ***7,42*** |

### EXEMPLE 7 : 1-(2-{4-[2-(p-Fluorophénoxy)éthyl]-1-pipéridinyl}éthyl)-3-(p-hydroxyphényl)-2-imidazolidinone et son chlorhydrate

Le produit est obtenu selon le procédé de l'exemple 5 en utilisant le produit de la préparation 25 à la place de celui de la préparation 23.
***Point de fusion*** ***(M.K.) : 201-206°C***

| ***Microanalyse élémentaire*** **:** | | | | |
|---|---|---|---|---|
| | ***C*** | ***H*** | ***N*** | ***Cl*** |
| ***% trouvé*** | ***61,82*** | ***6,73*** | ***8,94*** | ***7,65*** |
| ***% calculé*** | ***62,13*** | ***6,73*** | ***9,06*** | ***7,64*** |

### EXEMPLE 8 : 1-(2-{4-[2-(p-Fluorophénoxy)éthyl]-1-pipéridinyl}éthyl)-3(o-chlorophénoxy)-2-imidazolidinone et son chlorhydrate

Le produit est obtenu selon le procédé de l'exemple 5 en utilisant le produit de la préparation 26 à la place de celui de la préparation 23. Le produit est transformé en chlorhydrate par l'action d'une solution d'acide chlorhydrique dans l'acétonitrile.
***Point de fusion*** ***(M.K.) : 183-187°C***

| ***Microanalyse élémentaire*** **:** | | | | |
|---|---|---|---|---|
| | ***C*** | ***H*** | ***N*** | ***Cl*** |
| ***% trouvé*** | ***59,70*** | ***6,23*** | ***8,74*** | ***14,89*** |
| ***% calculé*** | ***59,75*** | ***6,27*** | ***8,71*** | ***14,70*** |

### EXEMPLE 9 : 1-{2-[4-(Phénoxyméthyl)-1-pipéridinyl]éthyl}-3-(p-hydroxyphényl)-2-imidazolidinone et son chlorhydrate

Le produit est obtenu selon le procédé de l'exemple 5 en utilisant comme substrat les composés des préparations 3 et 25. Le produit obtenu est transformé en son chlorhydrate par action d'une solution d'éther chlorhydrique.
***Point de fusion*** ***(M.K.) : 260-263°C***

| ***Microanalyse élémentaire*** **:** | | | | |
|---|---|---|---|---|
| | ***C*** | ***H*** | ***N*** | ***Cl*** |
| ***% trouvé*** | ***63,76*** | ***7,05*** | ***9,46*** | ***8,23*** |
| ***% calculé*** | ***63,95*** | ***7,00*** | ***9,73*** | ***8,23*** |

### EXEMPLE 10 : 1-{2-[4-(p-Fluorophénoxyméthyl)-1-pipéridinyl]éthyl}-3-(p-méthoxyphényl)-2-imidazolidinone et son chlorhydrate

Le produit est obtenu selon le procédé de l'exemple 5 en utilisant comme substrat les composés des préparations 1 et 24. Le produit obtenu est transformé en son chlorhydrate par action d'une solution d'éther chlorhydrique.
***Point de fusion*** ***(M.K.) : 287-290°C***

| ***Microanalyse élémentaire*** **:** | | | | |
|---|---|---|---|---|
| | ***C*** | ***H*** | ***N*** | ***Cl*** |
| ***% trouvé*** | ***62,28*** | ***6,93*** | ***9,02*** | ***7,81*** |
| ***% calculé*** | ***62,13*** | ***6,73*** | ***9,06*** | ***7,64*** |

### EXEMPLE 11 : 1-(2-{4-[2-(p-Fluorophénoxy)éthyl]-1-pipéridinyl}éthyl)-3-(p-fluorophényl)-2-imidazolidinone et son chlorhydrate

Le produit est obtenu selon le procédé de l'exemple 5 en utilisant comme substrat les composés des préparations 2 et 27. Le produit obtenu est transformé en son chlorhydrate par action d'une solution d'éther chlorhydrique.
***Point de fusion*** ***(M.K.) : 174-178°C***

| ***Microanalyse élémentaire*** **:** | | | | |
|---|---|---|---|---|
| | ***C*** | ***H*** | ***N*** | ***Cl*** |
| ***% trouvé*** | ***61,62*** | ***6,61*** | ***8,91*** | ***7,92*** |
| ***% calculé*** | ***61,86*** | ***6,49*** | ***9,02*** | ***7,61*** |

### EXEMPLE 12 : 1-{2-[4-(p-Fluorophénoxyméthyl)-1-pipéridinyl]éthyl}-3-(p-benzyloxyphényl)-2-imidazolidinone et son chlorhydrate

Le produit est obtenu selon le procédé de l'exemple 5 en utilisant comme substrat les composés des préparations 1 et 28. Le produit obtenu est transformé en son chlorhydrate par action d'une solution d'éther chlorhydrique.
***Point de fusion*** ***(M.K.) : 248-252°C***

| ***Microanalyse élémentaire*** **:** | | | | |
|---|---|---|---|---|
| | ***C*** | ***H*** | ***N*** | ***Cl*** |
| ***% trouvé*** | ***66,76*** | ***6,49*** | ***7,72,*** | ***6,75*** |
| ***% calculé*** | ***66,72*** | ***6,53*** | ***7,78*** | ***6,56*** |

### EXEMPLE 13 : 1-{2-[4-(p-Fluorophénoxyméthyl)-1-pipéridinyl]éthyl}-3-(p-hydroxyphényl)-2-imidazolidinone et son chlorhydrate

Le produit est obtenu selon le procédé de l'exemple 5 en utilisant comme substrat les composés des préparations 1 et 25. Le produit obtenu est transformé en son chlorhydrate par action d'une solution d'éther chlorhydrique.
***Point de fusion*** ***(M.K.) : 228-232°C***

| ***Microanalyse élémentaire*** **:** | | | | |
|---|---|---|---|---|
| | ***C*** | ***H*** | ***N*** | ***Cl*** |
| ***% trouvé*** | ***61,38*** | ***6,43*** | ***9,40*** | ***8,01*** |
| ***% calculé*** | ***61,40*** | ***6,50*** | ***9,34*** | ***7,88*** |

### EXEMPLE 14 : 1-{2-[4-(p-Méthylsulfonylphénoxyméthyl)-1-pipéridinyl]éthyl}-3-(p-hydroxyphényl)-2-imidazolidinone et son chlorhydrate

Le produit est obtenu selon le procédé de l'exemple 5 en utilisant comme substrat les composés des préparations 4 et 25. Le produit obtenu est transformé en son chlorhydrate par action d'une solution d'éther chlorhydrique.
***Point de fusion*** ***(M.K.) : 265-270°C***

| ***Microanalyse élémentaire*** **:** | | | | |
|---|---|---|---|---|
| | ***C*** | ***H*** | ***N*** | ***Cl*** |
| ***% trouvé*** | ***56,19*** | ***6,38*** | ***8,04*** | ***5,89*** |
| ***% calculé*** | ***56,52*** | ***6,32*** | ***8,24*** | ***6,29*** |

### EXEMPLE 15 : 1-(2-{4-[2-(p-Fluorophénoxy)éthyl]-1-pipéridinyl}éthyl)-3-(p-nitrophényl)-2-imidazolidinone et son chlorhydrate

Le produit est obtenu selon le procédé de l'exemple 5 en utilisant comme substrat les composés des préparations 2 et 29. Le produit obtenu est précipité sous forme de son chlorhydrate qui recristallise de l'acétonitrile.
***Point de fusion*** ***(M.K.) : 202-270°C***

| ***Microanalyse élémentaire*** **:** | | | | |
|---|---|---|---|---|
| | ***C*** | ***H*** | ***N*** | ***Cl*** |
| ***% trouvé*** | ***58,55*** | ***6,12*** | ***11,34*** | ***7,19*** |
| ***% calculé*** | ***58,47*** | ***6,13*** | ***11,36*** | ***7,19*** |

### EXEMPLE 16 : 1-(2-{4-[2-(p-Fluorophénoxy)éthyl]-1-pipéridinyl}éthyl)-3-(p-chlorophényl)-2-imidazolidinone et son chlorhydrate

Le produit est obtenu selon le procédé de l'exemple 5 en utilisant comme substrat les composés des préparations 2 et 30. Le produit obtenu est précipité sous forme de chlorhydrate qui recristallise de l'acétonitrile.
***Point de fusion*** ***(M.K.) : 180-184°C***

| ***Microanalyse élémentaire*** **:** | | | | |
|---|---|---|---|---|
| | ***C*** | ***H*** | ***N*** | ***Cl*** |
| ***% trouvé*** | ***59,97*** | ***6,23*** | ***8,68*** | ***14,95*** |
| ***% calculé*** | ***59,75*** | ***6,27*** | ***8,71*** | ***14,70*** |

### EXEMPLE 17 : 1-{2-[4-(p-Fluorophénoxyméthyl)-1-pipéridinyl]éthyl]-3-(p-nitrophényl)-2-imidazolidinone et son chlorhydrate

Le produit est obtenu selon le procédé de l'exemple 5 en utilisant comme substrat les composés des préparations 1 et 29. Le produit obtenu est précipité sous forme de son chlorhydrate et recristallisé de l'acétonitrile.
***Point de fusion*** ***(M.K.) : 205-209°C***

| ***Microanalyse élémentaire*** **:** | | | | |
|---|---|---|---|---|
| | ***C*** | ***H*** | ***N*** | ***Cl*** |
| ***% trouvé*** | ***57,83*** | ***5,89*** | ***11,60*** | ***6,87*** |
| ***% calculé*** | ***57,68*** | ***5,89*** | ***11,70*** | ***7,40*** |

### EXEMPLE 18 : 1-(2-{4-[2-(p-Méthylsulfonylphénoxy)éthyl]-1-pipéridinyl}éthyl)-3-(p-hydroxyphényl)-2-imidazolidinone et son chlorhydrate

Le produit est obtenu selon le procédé de l'exemple 5 en utilisant comme substrat les composés des préparations 5 et 25. Le produit obtenu est transformé en son chlorhydrate par action d'une solution d'éther chlorhydrique.
***Point de fusion*** ***(M.K.) : 120-125°C***

| ***Microanalyse élémentaire*** **:** | | | | | |
|---|---|---|---|---|---|
| | ***C*** | ***H*** | ***N*** | ***Cl*** | ***S*** |
| ***% trouvé*** | ***57,77*** | ***6,77*** | ***7,81*** | ***6,86*** | ***5,69*** |
| ***% calculé*** | ***57,30*** | ***6,54*** | ***8,02*** | ***6,76*** | ***6,12*** |

### EXEMPLE 49 : 1-(4-Fluorophényl)-3-{2-[4-(phénoxyméthyl)-1-pipéridinyl]éthyl}-2-imidazolidinone et son chlorhydrate

Le produit est obtenu selon le procédé de l'exemple 5 en utilisant comme substrats les composés des préparations 3 et 27. Le produit est transformé en son chlorhydrate par action d'une solution d'éther chlorhydrique.
***Point de fusion*** ***(M.K.) : 239-243°C***

| ***Microanalyse élémentaire*** **:** | | | | |
|---|---|---|---|---|
| | ***C*** | ***H*** | ***N*** | ***Cl*** |
| ***% trouvé*** | ***63,75*** | ***6,79*** | ***9,57*** | ***8,37*** |
| ***% calculé*** | ***63,65*** | ***6,74*** | ***9,69*** | ***8,17*** |

### EXEMPLE 50 : 1-(2-{4-[2-(4-Chlorophénoxy)éthyl]-1-pipéridinyl}éthyl)-3-(4-fluorophényl)-2-imidazolidinone et son chlorhydrate

Le produit est obtenu selon le procédé de l'exemple 5 en utilisant comme substrats les composés des préparations 31 et 27. Le produit est transformé en son chlorhydrate par action d'une solution d'éther chlorhydrique.
***Point de fusion*** ***(M.K.) : 189-193°C***

| ***Microanalyse élémentaire*** **:** | | | | |
|---|---|---|---|---|
| | ***C*** | ***H*** | ***N*** | ***Cl*** |
| ***% trouvé*** | ***59,31*** | ***6,12*** | ***8,61*** | ***14,74*** |
| ***% calculé*** | ***59,75*** | ***6,27*** | ***8,71*** | ***14,70*** |

### EXEMPLE 51 : 1-(4-Fluorophényl)-3-[2-(4-{[4-(méthylthio)phénoxy]méthyl}-1-pipéridinyl)éthyl]-2-imidazolidinone et son chlorhydrate

Le produit est obtenu selon le procédé de l'exemple 5 en utilisant comme substrats les composés des préparations 32 et 27. Le produit est transformé en son chlorhydrate par action d'une solution d'éther chlorhydrique.
***Point de fusion*** ***(M.K.) : 240-245°C***

| ***Microanalyse élémentaire*** **:** | | | | | |
|---|---|---|---|---|---|
| | ***C*** | ***H*** | ***N*** | ***Cl*** | ***S*** |
| ***% trouvé*** | ***60,07*** | ***6,64*** | ***8,62*** | ***7,71*** | ***6,72*** |
| ***% calculé*** | ***60,05*** | ***6,51*** | ***8,75*** | ***7,39*** | ***6,68*** |

### EXEMPLE 52 : 1-(4-Fluorophényl)-3-[2-(4-{2-[4-(méthylthio)phénoxy]éthyl}-1-pipéridinyl)éthyl]-2-imidazolidinone et son chlorhydrate

Le produit est obtenu selon le procédé de l'exemple 5 en utilisant comme substrats les composés des préparations 33 et 27. Le produit est transformé en son chlorhydrate par action d'une solution d'éther chlorhydrique.
***Point de fusion*** ***(M.K.) : 218-223°C***

| ***Microanalyse élémentaire*** **:** | | | | | |
|---|---|---|---|---|---|
| | ***C*** | ***H*** | ***N*** | ***Cl*** | ***S*** |
| ***% trouvé*** | ***61,28*** | ***6,88*** | ***8,62*** | ***7,31*** | ***6,06*** |
| ***% calculé*** | ***60,77*** | ***6,73*** | ***8,51*** | ***7,18*** | ***6,49*** |

### EXEMPLE 53 : 1-(4-Chlorophényl)-3-{2-[4-(phénoxyméthyl)-1-pipéridinyl]éthyl}-2-imidazolidinone et son chlorhydrate

Le produit est obtenu selon le procédé de l'exemple 5 en utilisant comme substrats les composés des préparations 3 et 30. Le produit est transformé en son chlorhydrate par action d'une solution d'éther chlorhydrique.
***Point de fusion*** ***(M.K.) : 277-282°C***

| ***Microanalyse élémentaire*** **:** | | | | |
|---|---|---|---|---|
| | ***C*** | ***H*** | ***N*** | ***Cl*** |
| ***% trouvé*** | ***61,67*** | ***6,34*** | ***9,33*** | ***15,94*** |
| ***% calculé*** | ***61,33*** | ***6,49*** | ***9,33*** | ***15,74*** |

### EXEMPLE 54 : 1-(4-Chlorophényl)-3-{2-[4-(2-phénoxyéthyl)-1-pipéridinyl]éthyl}-2-imidazolidinone et son chlorhydrate

Le produit est obtenu selon le procédé de l'exemple 5 en utilisant comme substrats les composés des préparations 34 et 30. Le produit est transformé en son chlorhydrate par action d'une solution d'éther chlorhydrique.
***Point de fusion*** ***(M.K.) : 215-219°C***

| ***Microanalyse élémentaire*** **:** | | | | |
|---|---|---|---|---|
| | ***C*** | ***H*** | ***N*** | ***Cl*** |
| ***% trouvé*** | ***62,07*** | ***6,73*** | ***8,98*** | ***15,47*** |
| ***% calculé*** | ***62,07*** | ***6,68*** | ***9,05*** | ***15,27*** |

### EXEMPLE 55 : 1-(2-{4-[2-(4-Chlorophénoxy)éthyl]-1-pipéridinyl}éthyl)-3-(4-chlorophényl)-2-imidazolidinone et son chlorhydrate

Le produit est obtenu selon le procédé de l'exemple 5 en utilisant comme substrats les composés des préparations 31 et 30. Le produit est transformé en son chlorhydrate par action d'une solution d'éther chlorhydrique.
***Point de fusion*** ***(M.K.) : 200-205°C***

| ***Microanalyse élémentaire*** **:** | | | | |
|---|---|---|---|---|
| | ***C*** | ***H*** | ***N*** | ***Cl*** |
| ***% trouvé*** | ***57,97*** | ***5,94*** | ***8,45*** | ***21,52*** |
| ***% calculé*** | ***57,78*** | ***6,06*** | ***8,42*** | ***21,32*** |

### EXEMPLE 56 : 1-(4-Chlorophényl)-3-(2-{4-[(4-fluorophénoxy)méthyl]-1-pipéridinyl}éthyl)-2-imidazolidinone et son chlorhydrate

Le produit est obtenu selon le procédé de l'exemple 5 en utilisant comme substrats les composés des préparations 1 et 30. Le produit est transformé en son chlorhydrate par action d'une solution d'éther chlorhydrique.
***Point de fusion*** ***(M.K.) : 218-222°C***

| ***Microanalyse élémentaire*** **:** | | | | |
|---|---|---|---|---|
| | ***C*** | ***H*** | ***N*** | ***Cl*** |
| ***% trouvé*** | ***58,91*** | ***5,97*** | ***8,82*** | ***15,37*** |
| ***% calculé*** | ***58,97*** | ***6,03*** | ***8,97*** | ***15,14*** |

### EXEMPLE 57 : 1-(4-Chlorophényl)-3-[2-(4-{[4-(méthylthio)phénoxy] méthyl}-1-pipéridinyl)éthyl]-2-imidazolidinone et son chlorhydrate

Le produit est obtenu selon le procédé de l'exemple 5 en utilisant comme substrats les composés des préparations 32 et 30. Le produit est transformé en son chlorhydrate par action d'une solution d'éther chlorhydrique.
***Point de fusion*** ***(M.K.) : 288-292°C***

| ***Microanalyse élémentaire*** **:** | | | | | |
|---|---|---|---|---|---|
| | ***C*** | ***H*** | ***N*** | ***Cl*** | ***S*** |
| ***% trouvé*** | ***58,26*** | ***6,40*** | ***8,41*** | ***14,38*** | ***6,69*** |
| ***% calculé*** | ***58,06*** | ***6,29*** | ***8,46*** | ***14,28*** | ***6,46*** |

### EXEMPLE 58 : 1-(4-Nitrophényl)-3-{2-[4-(phénoxyméthyl)-1-pipéridinyl]éthyl}-2-imidazolidinone et son chlorhydrate

Le produit est obtenu selon le procédé de l'exemple 5 en utilisant comme substrats les composés des préparations 3 et 29. Le produit est transformé en son chlorhydrate par action d'une solution d'éther chlorhydrique.
***Point de fusion*** ***(M.K.) : 216-220°C***

| ***Microanalyse élémentaire*** **:** | | | | |
|---|---|---|---|---|
| | ***C*** | ***H*** | ***N*** | ***Cl*** |
| ***% trouvé*** | ***59,51*** | ***6,36*** | ***11,98*** | ***7,82*** |
| ***% calculé*** | ***59,93*** | ***6,34*** | ***12,15*** | ***7,69*** |

### EXEMPLE 59 : 1-[2-(4-{[4-(Méthylthio)phénoxy]méthyl}-1-pipéridinyl)éthyl]-3-(4-nitrophényl)-2-imidazolidinone et son chlorhydrate

Le produit est obtenu selon le procédé de l'exemple 5 en utilisant comme substrats les composés des préparations 32 et 29. Le produit est transformé en son chlorhydrate par action d'une solution d'éther chlorhydrique.
***Point de fusion*** ***(M.K.) : 243-247°C***

| ***Microanalyse élémentaire*** **:** | | | | | |
|---|---|---|---|---|---|
| | ***C*** | ***H*** | ***N*** | ***Cl*** | ***S*** |
| *% **trouvé*** | ***56,57*** | ***6,09*** | ***10,83*** | ***6,91*** | ***5,99*** |
| ***% calculé*** | ***56,85*** | ***6,16*** | ***11,05*** | ***6,99*** | ***6,32*** |

### EXEMPLE 60 : 1-(2-{4-[2-(4-Chlorophénoxy)éthyl]-1-pipéridinyl}éthyl)-3-(4-nitrophényl)-2-imidazolidinone et son chlorhydrate

Le produit est obtenu selon le procédé de l'exemple 5 en utilisant comme substrats les composés des préparations 31 et 29. Le produit est transformé en son chlorhydrate par action d'une solution d'éther chlorhydrique.
***Point de fusion*** ***(M.K.) : 196-201°C***

| ***Microanalyse élémentaire*** **:** | | | | |
|---|---|---|---|---|
| | ***C*** | ***H*** | ***N*** | ***Cl*** |
| ***% trouvé*** | ***57,02*** | ***5,88*** | ***10,91*** | ***14,26*** |
| ***% calculé*** | ***56,59*** | ***5,94*** | ***11,00*** | ***13,92*** |

### EXEMPLE 61 : 1-(4-Hydroxyphényl)-3-[2-(4-{[4-(méthylthio)phénoxy]méthyl}-1-pipéridinyl)éthyl]-2-imidazolidinone et son chlorhydrate

Le produit est obtenu selon le procédé de l'exemple 5 en utilisant comme substrats les composés des préparations 32 et 25. Le produit est transformé en son chlorhydrate par action d'une solution d'éther chlorhydrique.
***Point de fusion*** ***(M.K.) : 273-277°C***

| ***Microanalyse élémentaire*** **:** | | | | | |
|---|---|---|---|---|---|
| | ***C*** | ***H*** | ***N*** | ***Cl*** | ***S*** |
| ***% trouvé*** | ***60,07*** | ***6,72*** | ***8,78*** | ***7,09*** | ***6,34*** |
| ***% calculé*** | ***60,30*** | ***6,75*** | ***8,79*** | ***7,42*** | ***6,71*** |

### EXEMPLE 62 : 1-{2-[4-(Phénoxyméthyl)-1-pipéridinyl]éthyl}-3-[4- (trifluorométhyl)phényl]-2-imidazolidinone et son chlorhydrate

Le produit est obtenu selon le procédé de l'exemple 5 en utilisant comme substrats les composés des préparations 3 et 35. Le produit est transformé en son chlorhydrate par action d'une solution d'éther chlorhydrique.
***Point de fusion*** ***(M.K.) : 224-228°C***

| ***Microanalyse élémentaire*** **:** | | | | |
|---|---|---|---|---|
| | ***C*** | ***H*** | ***N*** | ***Cl*** |
| ***% trouvé*** | ***59,70*** | ***6,12*** | ***8,66*** | ***7,46*** |
| ***% calculé*** | ***59,56*** | ***6,04*** | ***8,68*** | ***7,33*** |

### EXEMPLE 63 : 4-(2-Oxo-3-{2-[4-(phénoxyméthyl)-1-pipéridinyl]éthyl}-1-imidazolidinyl)benzonitrile et son chlorhydrate

Le produit est obtenu selon le procédé de l'exemple 5 en utilisant comme substrats les composés des préparations 3 et 36. Le produit est transformé en son chlorhydrate par action d'une solution d'éther chlorhydrique.
***Point de fusion*** ***(M.K.) : 210-215°C***

| ***Microanalyse élémentaire*** **:** | | | | |
|---|---|---|---|---|
| | ***C*** | ***H*** | ***N*** | ***Cl*** |
| ***% trouvé*** | ***64,82*** | ***6,63*** | ***12,23*** | ***7,74*** |
| ***% calculé*** | ***65,37*** | ***6,63*** | ***12,71*** | ***8,04*** |

### EXEMPLE 64 : 1-(4-Fluoro-3-nitrophényl)-3-(2-{4-[2-(4-fluorophénoxy)éthyl]-1-pipéridinyl}éthyl)-1,3-dihydro-2H-imidazol-2-one et son chlorhydrate

Le produit est obtenu selon le procédé de l'exemple 1 des stades 1 à 2, en utilisant comme substrats les composés des préparations 37 et l'isocyanate de 4-fluoro-3-nitrophényle. Le produit est transformé en son chlorhydrate par action d'une solution d'éther chlorhydrique.
***Point de fusion*** ***(M.K.) : 205-209°C***

| ***Microanalyse élémentaire*** **:** | | | | |
|---|---|---|---|---|
| | ***C*** | ***H*** | ***N*** | ***Cl*** |
| ***% trouvé*** | ***56,58*** | ***5,23*** | ***10,95*** | ***7,06*** |
| *% **calculé*** | ***56,64*** | ***5,35*** | ***11,01*** | ***6,97*** |

### EXEMPLE 107 : 1-(4-Fluorophényl)-3-(2-{4-[2-(phénoxy)éthyl]-1-pipéridinyl}éthyl)-2-imidazolidinone et son chlorhydrate

Le produit est obtenu selon le procédé de l'exemple 5, en utilisant les composés des préparations 34 et 27. Le produit est transformé en son chlorhydrate par action d'une solution d'éther chlorhydrique.
***Point de fusion*** ***(M.K.) : 176-179°C***

### EXEMPLE 108 : 1-(4-Nitrophényl)-3-(2-{4-[2-(phénoxy)éthyl]-1-pipéridinyl}éthyl)-2-imidazolidinone et son chlorhydrate

Le produit est obtenu selon le procédé de l'exemple 5, en utilisant les composés des préparations 34 et 29. Le produit est transformé en son chlorhydrate par action d'une solution d'éther chlorhydrique.
***Point de fusion*** ***(M.K.) : 197-202°C***

### EXEMPLE 109 : 1-{2-[4-(Phénoxyméthyl)-1-pipéridinyl]éthyl}-3-phényl-2-imidazolidinone et son chlorhydrate

Le produit est obtenu selon le procédé de l'exemple 5, en utilisant le composé de la préparation 3 à la place de celui de la préparation 2. Le produit est transformé en son chlorhydrate par action d'une solution d'éther chlorhydrique.
***Point de fusion*** ***(M.K.) : 214-218°C***

### ETUDE PHARMACOLOGIQUE DES COMPOSES DE L'INVENTION

Dans des conditions standard *in vitro*, la relaxation à l'acétylcholine (ACh) d'anneaux aortiques, totalement dépendante de la présence de l'endothélium, est le reflet de la production de NO (stimulée par l'ACh) qui en diffusant au niveau des cellules musculaires lisses entraîne la relaxation artérielle (*Nature*, **1980,** 288, 373).
Les composés de l'invention ont été testés sur deux modèles mettant en jeu deux mécanismes différents impliqués dans la dysfonction endothéliale observée en pathologie :
- le premier modèle consiste à induire une inhibition de la relaxation à l'ACh par blocage de l'activité enzymatique (NOS endothéliale) responsable de la production de NO.
- le second modèle consiste à induire un stress oxydant *in vitro* par un système enzymatique générateur d'O₂⁻ (xanthine oxydase -XO et hypoxanthine -Hypo).

### EXEMPLE 111 : Effets protecteurs vasculaires vis-à-vis d'une dysfonction endothéliale induite par un inhibiteur de NOS

L'aorte thoracique de rat Wistar (325-375 g), anesthésié par voie intrapérinétonéale au pentobarbital sodique (30 mg/kg), est prélevée et disséquée en anneaux de 3 mm de longueur. Chaque anneau est suspendu à un capteur de tension isométrique connecté à un système d'acquisition et la tension initiale appliquée est de 2,5 g. La solution physiologique utilisée, thermostatée à 37°C et oxygénée (95 % O₂ + 5 % CO₂) est composée de (en mM) : NaCl 112,0 ; KCl 5,0 ; CaCl₂ 2,5 ; KH₂PO₄ 1,0 ; MgSO₄ 1,2 ; NaHCO₃ 25,0 ; glucose 11,5, Ca-EDTA 0,016.
Après une période de stabilisation de 90 minutes, les préparations sont contractées avec de la phényléphrine (PHE 10⁻⁶M) et relaxées par addition de 10⁻⁵M d'acétylcholine afin de vérifier l'intégrité de la couche endothéliale. Si celle-ci est confirmée, les préparations sont rincées et une concentration de produit à tester est additionnée au milieu suivie de 3.10⁻⁷M de N^{G}-niro-L-arginine (LNA). Les préparations sont à nouveau contractées avec de la phényléphrine et 30 minutes après les relaxations à l'acétylcholine (ACh-10⁻⁸M à 10⁻⁵M) sont évaluées en présence d'indométhacine (10⁻⁵M).
Les valeurs de relaxation sont exprimées en pourcentage par rapport à la contraction maximale à la PHE. Les effets protecteurs des composés vis-à-vis de la dysfonction endothéliale correspondent à la différence entre les pourcentages de relaxation maximale observée en présence ou en absence de produit.

### EXEMPLE 112 : Effets protecteurs vasculaires vis-à-vis d'une dysfonction endothéliale induite par un système générateur de O₂⁻

Ce protocole, réalisé sur anneaux aortiques de lapins Néo-Zélandais (2,5-3 kg) est comparable au précédent hormis pour les points suivants : la tension initiale appliquée est de 5 g et l'association XO (3 mU/ml -Hypo (10⁻⁴M) est utilisée à la place du LNA.

### EXEMPLE 113 : Implication de la voie du NO dans les effets protecteurs vasculaires détectés : évaluation de la production aortique de GMPc

En diffusant au niveau des cellules musculaires lisses, le NO produit par les cellules endothéliales active la guanylate cyclase soluble ce qui entraîne une augmentation du GMP cyclique responsable de la relaxation.
Ce médiateur a donc été dosé sur les anneaux aortiques de rat afin de démontrer que les effets protecteurs des composés vis-à-vis de la dysfonction endothéliale sont médiés par une augmentation de la disponibilité en NO.
Les anneaux aortiques de rat sont préparés comme précédemment. Les effets d'une incubation de 30 minutes des composés de l'invention à différentes concentrations sont évalués sur la production de GMPc stimulée par l'ACh (10⁻⁵M - 1 minute) en présence de LNA (3x10⁻⁶M). Ces expériences sont réalisées en présence d'isobutyl méthyl xanthine (10⁻⁵M) afin d'éviter la dégradation du GMPc par les phosphodiestérases. Les anneaux sont congelés dans de l'azote liquide et maintenus à -80°C jusqu'au dosage. Le contenu en GMPc est évalué par radio-immunodosage et exprimé par rapport à la quantité de protéines contenues dans le tissu (dosage par la méthode de Bradford).

## Revendications

1. Composés de formule (I), **caractérisés en ce qu'**ils représentent des composés de formule (IA) : 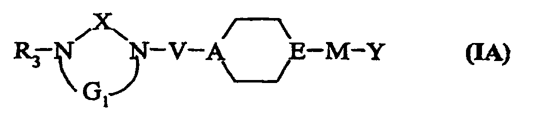 dans laquelle :
R₃ représente un groupement aryle, éventuellement substitué par un groupement choisi parmi atome d'halogène, groupement hydroxy, alkoxy (C₁-C₆) linéaire ou ramifié, arylalkoxy (C₁-C₆) linéaire ou ramifié, nitro, trihalogénoalkyle (C₁-C₆) linéaire ou ramifié, et cyano,
X représente un groupement carbonyle,
G₁ représente une chaîne alkylène (C₂-C₃) linéaire,
V représente une chaine alkylone (C₁-C₆) linéaire ou ramifiée,
A représente un atome d'azote quand E représente un groupement CH, ou
A représente un groupement CH quand E représente un atome d'azote,
M représente une chaîne alkylène (C₁-C₄) linéaire ou ramifiée,
Y représente un groupement benzofuran-3-yl ou un groupement phényloxy éventuellement substitué par un groupement choisi parmi atome d'halogène, groupement alkylsulfonyle (C₁-C₆) linéaire ou ramifié, et alkylthio (C₁-C₆) linéaire ou ramifié,
leurs isomères ainsi que leurs hydrates, leurs solvates et leurs sels d'addition à un acide pharmaceutiquement acceptable,
étant entendu que par groupement aryle, on comprend un groupement choisi parmi phényle, biphényle, naphtyle, dihydronaphtyle, tétrahydronaphtyle, indanyle, indényle, et benzocyclobutyle.

2. Composés de formule (IA) selon la revendication 1 **caractérisés en ce que** R₃ représente un groupement phényle éventuellement substitué par un atome d'halogène, leurs isomères ainsi que leurs hydrates, leurs solvates et leurs sels d'addition à un acide pharmaceutiquement acceptable.

3. Composés de formule (IA) selon la revendication 1 **caractérisés en ce que** :
* A représente un atome d'azote et E représente un groupement CH quand Y représente un groupement phényloxy éventuellement substitué,
* ou A représente un groupement CH et E représente un atome d'azote quand Y représente un groupement benzofuran-3-yl,
leurs isomères ainsi que leurs hydrates, leurs solvates et leurs sels d'addition à un acide pharmaceutiquement acceptable.

4. Composés de formule (IA) selon la revendication 1 **caractérisés en ce que** Y représente un groupement phényloxy éventuellement substitué,
leurs isomères ainsi que leurs hydrates, leurs solvates et leurs sels d'addition à un acide pharmaceutiquement acceptable.

5. Composé de formule (I) selon la revendication 1 qui est la 1-(2-{4-[2-(4-fluorophénoxy)éthyl]-1-pipéridinyl}éthyl)-3-phényl-2-imidazolidinone, ses isomères ainsi que ses hydrates, ses solvates et ses sels d'addition à un acide pharmaceutiquement acceptable.

6. Procédé de préparation des composés de formule (I) selon la revendication 1 **caractérisé en ce qu'**on utilise comme produit de départ un composé de formule (II):
R₃ - N = C = O **(II)**
dans laquelle R₃ est tel que défini dans la formule (I),
que l'on fait réagir :
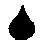 soit avec un composé de formule (III) :
HO - G₁ₐ - NH - Vₐ - OH **(III)**
dans laquelle Vₐ représente une chaîne alkylène (C₂-C₆) et G₁ₐ représente une chaîne alkylène linéaire en (C₂-C₃),
pour conduire aux composés de formule (IVa) : 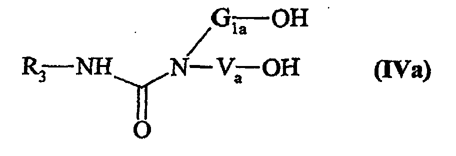 dans laquelle R₃, G₁ₐ, et Vₐ sont tels que définis précédemment,
composés de formule (IVa) qui sont transformés par les méthodes classiques de la synthèse organique, pour conduire aux composés de formule (IVb) : 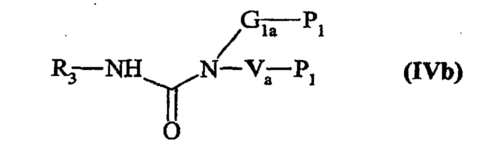 dans laquelle P₁ représente un atome de chlore, de brome, d'iode, ou un groupement OSO₂R₄ dans lequel R₄ représente un groupement méthyle, trifluorométhyle ou tolyle, R₃, Vₐ et G₁ₐ sont tels que définis précédemment,
composés de formule (IVb) que l'on soumet à l'action de la chaleur,
pour conduire aux composés de formule (IVc) : 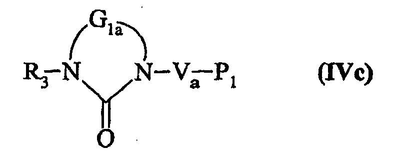 dans laquelle R₃, G₁ₐ, Vₐ et P₁ sont tels que définis précédemment, composés de formule (IVc) qui sont traités en condition basique par un composé de formule (V) : 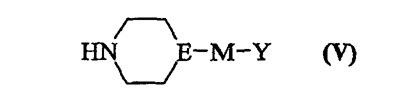 dans laquelle E, M et Y sont tels que définis dans la formule (I),
pour conduire aux composés de formule (I/a), cas particulier des composés de formule (I) : 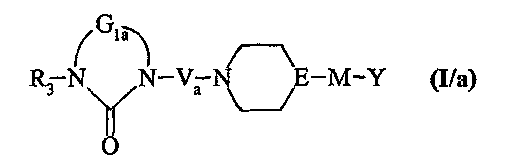 dans laquelle R₃, G₁ₐ, Vₐ, E, M et Y sont tels que définis précédemment,
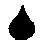 soit avec un composé de formule (IX) : 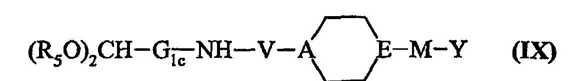 dans laquelle G_{1c} représente une chaîne alkylène linéaire en C₁ ou C₂, R₅ représente un groupement alkyle (C₁-C₆) linéaire ou ramifié, V, A, E, M et Y sont tels que définis dans la formule (I),
pour conduire aux composés de formule (X) : 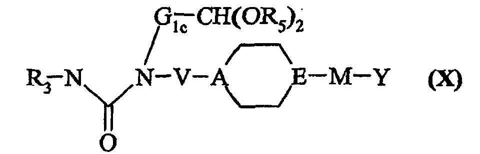 dans laquelle R₃, G_{1c}, V, A, R₅, M et Y sont tels que définis précédemment, composés de formule (X) que l'on place en présence d'acide organique fort,
pour conduire aux composés de formule (I/c) : 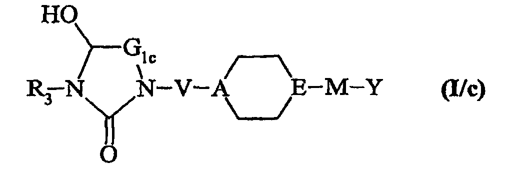 dans laquelle R₃, G_{1c}, V, A, E, M et Y sont tels que définis précédemment, composé de formule (I/c) que l'on hydrogène selon des techniques classiques de la synthèse organique,
pour conduire aux composés de formule (I/d), cas particulier des composés de formule (I) : 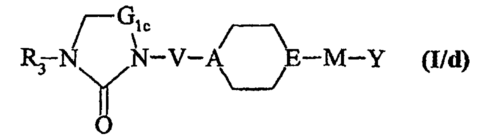 dans laquelle R₃, G_{1c}, V, A, E, M et Y sont tels que définis précédemment,
l'ensemble des composés de formule (I/a) et (I/d) forment les composés de l'invention, que l'on purifie, le cas échéant, selon des techniques classiques de purification, dont on sépare éventuellement les isomères selon une technique classique de séparation, et que l'on transforme, si on le souhaite, en ses hydrates, ses solvates ou ses sels d'addition à un acide pharmaceutiquement acceptable.

7. Compositions pharmaceutiques contenant comme principe actif au moins un composé de formule (I) selon l'une quelconque des revendications 1 à 5, seul ou en combinaison avec un ou plusieurs excipients ou véhicules inertes, non toxiques, pharmaceutiquement acceptables.

8. Compositions pharmaceutiques selon la revendication 7 contenant au moins un principe actif selon l'une quelconque des revendications 1 à 5, utiles en tant que médicament pour le traitement des maladies ou des conditions pathologiques dans lesquelles une dysfonction endothéliale est connue.

9. Compositions pharmaceutiques selon la revendication 7 contenant au moins un principe actif selon l'une quelconque des revendications 1 à 5, utiles en tant que médicament pour prévenir le développement, l'extension et les complications des lésions athéroscléreuses, en particulier chez les patients présentant un facteur de risque vasculaire (dyslipidémie, diabète, hypertension artérielle systémique), pour prévenir les complications vasculaires après pontage vasculaire, dilatation vasculaire, reperméabilisation vasculaire et transplantation cardiaque, ou pour traiter l'ischémie myocardique ou périphérique, l'insuffisance cardiaque et l'hypertension artérielle pulmonaire.

## Patentansprüche

1. Verbindungen der Formel (I), **dadurch gekennzeichnet, daß** sie die Verbindungen der Formel (IA) darstellen: 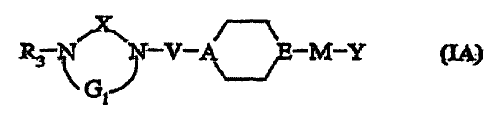 in der:
R₃ eine Arylgruppe bedeutet, die gegebenenfalls durch eine Gruppe ausgewählt aus Halogenatomen, Hydroxy-, geradkettigen oder verzweigten (C₁-C₆)-Alkoxy-, geradkettigen oder verzweigten Aryl-(C₁-C₆)-alkoxy-, Nitro-, geradkettigen oder verzweigten (C₁-C₆)-Trihalogenalkyl- und Cyanogruppen substituiert ist,
X eine Carbonylgruppe bedeutet,
G₁ eine geradkettige (C₂-C₃)-Alkylenkette bedeutet,
V eine geradkettige oder verzweigte (C₁-C₆)-Alkylenkette bedeutet,
A ein Stickstoffatom bedeutet, wenn E eine Gruppe CH darstellt, oder
A eine Gruppe CH bedeutet, wenn E ein Stickstoffatom darstellt,
M eine geradkettige oder verzweigte (C₁-C₄)-Alkylenkette bedeutet,
Y eine Benzofuran-3-yl-gruppe oder eine Phenyloxygruppe, die gegebenenfalls durch eine Gruppe ausgewählt aus Halogenatomen, geradkettigen oder verzweigten (C₁-C₆)-Alkylsulfonylgruppen und geradkettigen oder verzweigten (C₁-C₆)-Alkylthiogruppen substituiert ist, bedeutet,
deren Isomere sowie deren Hydrate, deren Solvate und deren Additionssalze mit einer pharmazeutisch annehmbaren Säure,
mit der Maßgabe, daß man unter einer Arylgruppe eine Gruppe versteht, ausgewählt aus Phenyl, Biphenyl, Naphthyl, Dihydronaphthyl, Tetrahydronaphthyl, Indanyl, Indenyl und Benzocyclobutyl.

2. Verbindungen der Formel (IA) nach Anspruch 1, **dadurch gekennzeichnet, daß** R₃ eine Phenylgruppe bedeutet, die gegebenenfalls durch ein Halogenatom substituiert ist, deren Isomere sowie deren Hydrate, deren Solvate und deren Additionssalze mit einer pharmazeutisch annehmbaren Säure.

3. Verbindungen der Formel (IA) nach Anspruch 1, **dadurch gekennzeichnet, daß**:
* A ein Stickstoffatom und E eine Gruppe CH bedeuten, wenn Y eine gegebenenfalls substituierte Phenyloxygruppe darstellt,
* oder A eine Gruppe CH und E ein Stickstoffatom bedeuten, wenn Y eine Benzofuran-3-yl-gruppe darstellt,
deren Isomere sowie deren Hydrate, deren Solvate und deren Additionssalze mit einer pharmazeutisch annehmbaren Säure.

4. Verbindungen der Formel (IA) nach Anspruch 1, **dadurch gekennzeichnet, daß** Y eine gegebenenfalls substituierte Phenyloxygruppe bedeutet,
deren Isomere sowie deren Hydrate, deren Solvate und deren Additionssalze mit einer pharmazeutisch annehmbaren Säure.

5. Verbindung der Formel (IA) nach Anspruch 1, nämlich 1-(2-{4-[2-(4-Fluorphenoxy)-ethyl]-1-piperidinyl}-ethyl)-3-phenyl-2-imidazolidinon, dessen Isomere sowie dessen Hydrate, dessen Solvate und dessen Additionssalze mit einer pharmazeutisch annehmbaren Säure.

6. Verfahren zur Herstellung der Verbindungen der Formel (I) nach Anspruch 1, **dadurch gekennzeichnet, daß** man als Ausgangsprodukt eine Verbindung der Formel (II) verwendet:
R₃ - N = C = O **(II)**
in der R₃ die bezüglich der Formel (I) angegebenen Bedeutungen besitzt, welche man:
• entweder mit einer Verbindung der Formel (III) umsetzt:
HO - G₁ₐ - NH - Vₐ - OH **(III)**
in der Vₐ eine (C₂-C₆)-Alkylenkette und G₁ₐ eine geradkettige (C₂-C₃)-Alkylenkette bedeuten,
zur Bildung der Verbindungen der Formel (IVa): 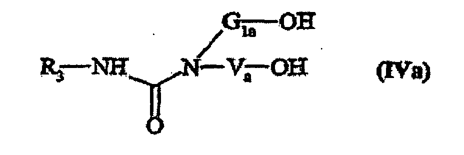 in der R₃, G₁ₐ und Vₐ die oben angegebenen Bedeutungen besitzen,
welche Verbindungen der Formel (IVa) man mit Hilfe klassischer Methoden der organischen Synthese umwandelt zur Bildung der Verbindungen der Formel (IVb): 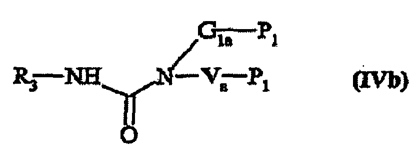 in der P₁ ein Chlor-, Brom- oder Iodatom oder eine Gruppe OSO₂R₄ darstellt, in der R₄ eine Methyl-, Trifluormethyl- oder Tolylgruppe bedeutet und R₃, Vₐ und G₁ₐ die oben angegebenen Bedeutungen besitzen,
welche Verbindungen der Formel (IVb) man der Einwirkung von Wärme unterwirft, zur Bildung der Verbindungen der Formel (IVc): 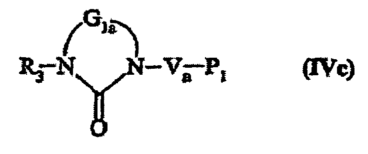 in der R₃, G₁ₐ, Vₐ und P₁ die oben angegebenen Bedeutungen besitzen, welche Verbindungen der Formel (IVc) man unter basischen Bedingungen mit einer Verbindung der Formel (V) behandelt: 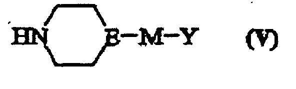 in der E, M und Y die bezüglich der Formel (I) angegebenen Bedeutungen besitzen, zur Bildung der Verbindungen der Formel (I/a), einem Sonderfall der Verbindungen der Formel (I): 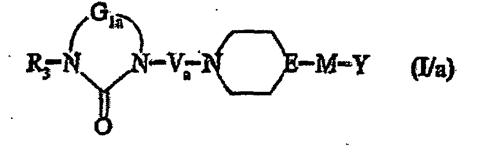 in der R₃, G₁ₐ, Vₐ, E, M und Y die oben angegebenen Bedeutungen besitzen,
• oder mit einer Verbindung der Formel (IX) umsetzt: 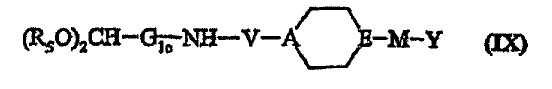 in der G_{1c} eine geradkettige C₁- oder C₂-Alkylenkette und R₅ eine geradkettige oder verzweigte (C₁-C₆)-Alkylgruppe bedeuten und V, A, E, M und Y die bezüglich der Formel (I) angegebenen Bedeutungen besitzen,
zur Bildung der Verbindungen der Formel (X): 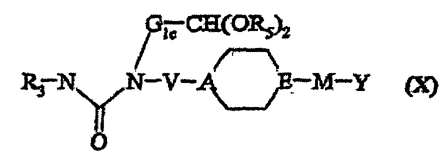 in der R₃, G_{1c}, V, A, R₅, M und Y die oben angegebenen Bedeutungen besitzen, welche Verbindungen der Formel (X) man mit einer starken organischen Säure in Kontakt bringt,
zur Bildung der Verbindungen der Formel (I/c): 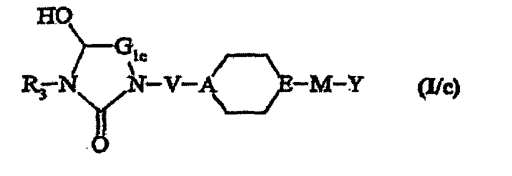 in der R₃, G_{1c}, V, A, E, M und Y die oben angegebenen Bedeutungen besitzen, welche Verbindung der Formel (I/c) man mit Hilfe klassischer Methoden der organischen Synthese hydriert,
zur Bildung der Verbindungen der Formel (I/d), einem Sonderfall der Verbindungen der Formel (I): 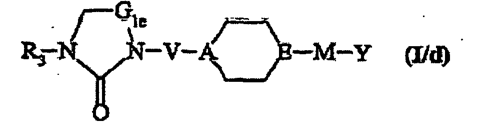 in der R₃, G_{1c}, V, A, E, M und Y die oben angegebenen Bedeutungen besitzen,
wobei die Gesamtheit der Verbindungen der Formeln ((I/a) und (I/d) die erfindungsgemäßen Verbindungen bildet, die man gegebenenfalls mit Hilfe klassischer Reinigungsmethoden reinigt, die man gegebenenfalls mit Hilfe einer klassischen Trennmethode in ihre Isomeren auftrennt und die man gewünschtenfalls in ihre Hydrate, ihre Solvate oder ihre Additionssalze mit einer pharmazeutisch annehmbaren Säure überführt.

7. Pharmazeutische Zubereitungen, enthaltend als Wirkstoff mindestens eine Verbindung der Formel (I) nach einem der Ansprüche 1 bis 5 allein oder in Kombination mit einem oder mehreren inerten, nichttoxischen, pharmazeutisch annehmbaren Trägermaterialien oder Bindemitteln.

8. Pharmazeutische Zubereitungen nach Anspruch 7, enthaltend mindestens einen Wirkstoff nach einem der Ansprüche 1 bis 5, geeignet als Arzneimittel für die Behandlung von Krankheiten oder pathologischen Zuständen, bei denen eine endotheliale Dysfunktion bekannt ist.

9. Pharmazeutische Zubereitungen nach Anspruch 7, enthaltend mindestens einen Wirkstoff nach einem der Ansprüche 1 bis 5, geeignet als Arzneimittel zur Vorbeugung der Entwicklung, der Ausbreitung und der Komplikationen von atherosklerösen Schädigungen, insbesondere bei Patienten, die einen Gefäßrisikofaktor aufweisen (Dyslipidämie, Diabetes, arterielle systemische Hypertension), zur Vorbeugung von Gefäßkomplikationen nach der Gefäßüberbrückung, der Gefäßerweiterung, dem Durchgängigmachen der Gefäße und von Herztransplantationen oder zur Behandlung von myokardischer oder peripherer Ischämie, von Herzinsuffizienz und pulmonalarterieller Hypertension.

## Claims

1. Compounds of formula (I), **characterised in that** they represent compounds of formula (IA) : 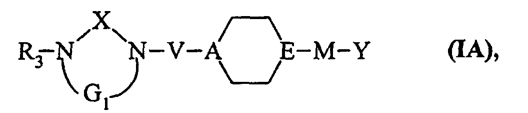 wherein :
R₃ represents an aryl group, optionally substituted by a group selected from a halogen atom, a hydroxy group, a linear or branched (C₁-C₆)alkoxy group, an aryl-(C₁-C₆)-alkoxy group in which the alkoxy moiety is linear or branched, a nitro group, a linear or branched (C₁-C₆)trihaloalkyl group and a cyano group,
X represents a carbonyl group,
G₁ represents a linear (C₂-C₃)alkylene chain,
V represents a linear or branched (C₁-C₆)alkylene chain,
A represents a nitrogen atom when E represents a CH group, or
A represents a CH group when E represents a nitrogen atom,
M represents a linear or branched (C₁-C₄)alkylene chain,
Y represents a benzofuran-3-yl group or a phenyloxy group optionally substituted by a group selected from a halogen atom, a linear or branched (C₁-C₆)alkylsulphonyl group and a linear or branched (C₁-C₆)alkylthio group,
their isomers, and also their hydrates, their solvates and addition salts thereof with a pharmaceutically acceptable acid,
an aryl group being understood to mean a group selected from phenyl, biphenyl, naphthyl, dihydronaphthyl, tetrahydronaphthyl, indanyl, indenyl and benzocyclobutyl.

2. Compounds of formula (IA) according to claim 1, **characterised in that** R₃ represents a phenyl group optionally substituted by a halogen atom, their isomers, and also their hydrates, their solvates and addition salts thereof with a pharmaceutically acceptable acid.

3. Compounds of formula (IA) according to claim 1, **characterised in that**:
* A represents a nitrogen atom and E represents a CH group when Y represents an optionally substituted phenyloxy group,
* or A represents a CH group and E represents a nitrogen atom when Y represents a benzofuran-3-yl group,
their isomers, and also their hydrates, their solvates and addition salts thereof with a pharmaceutically acceptable acid.

4. Compounds of formula (IA) according to claim 1, **characterised in that** Y represents an optionally substituted phenyloxy group,
their isomers, and also their hydrates, their solvates and addition salts thereof with an pharmaceutically acceptable acid.

5. Compound of formula (I) according to claim 1 which is 1-(2-{4-[2-(4-fluorophenoxy)ethyl]-1-piperidyl}ethyl)-3-phenyl-2-imidazolidinone, its isomers, and also its hydrates, its solvates and addition salts thereof with a pharmaceutically acceptable acid.

6. Process for the preparation of compounds of formula (I) according to claim 1, **characterised in that** there is used as starting material a compound of formula (II) :
R₃ - N = C = O **(II),**
wherein R₃ is as defined for formula (I),
which is reacted :
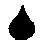 either with a compound of formula (III):
HO - G₁ₐ - NH - Vₐ - OH **(III),**
wherein Vₐ represents a (C₂-C₆)alkylene chain and G₁ₐ represents a linear (C₂-C₃)alkylene chain,
to yield the compounds of formula (IVa): 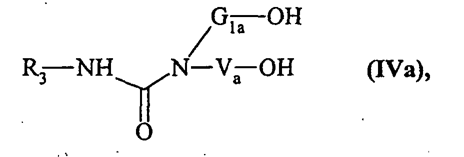 wherein R₃, G₁ₐ and Vₐ are as defined hereinbefore,
which compounds of formula (IVa) are converted by conventional methods of organic synthesis to yield the compounds of formula (IVb) : 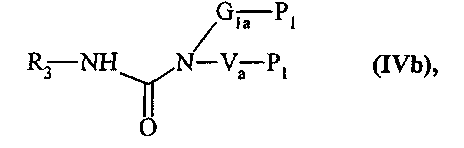 wherein P₁ represents a chlorine, bromine or iodine atom or a group OSO₂R₄ wherein R₄ represents a methyl, trifluoromethyl or tolyl group, and R₃, Vₐ and G₁ₐ are as defined hereinbefore,
which compounds of formula (IVb) are subjected to the action of heat
to yield the compounds of formula (IV c) : 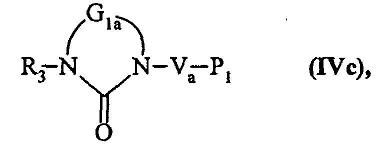 wherein R₃, G₁ₐ, Vₐ and P₁ are as defined hereinbefore, which compounds of formula (IVc) are treated under basic conditions with a compound of formula (V) : 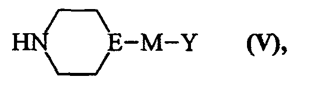 wherein E, M and Y are as defined for formula (I),
to yield the compounds of formula (I/a), a particular case of the compounds of formula (I) : 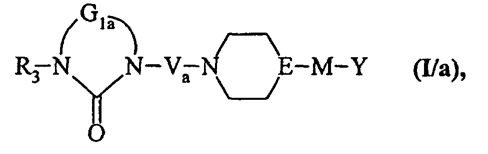 wherein R₃, G₁ₐ, Vₐ, E, M and Y are as defined hereinbefore,
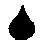 or with a compound of formula (IX) : 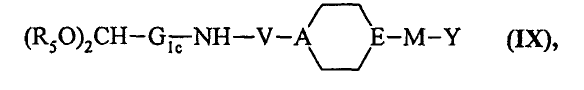 wherein G_{1c} represents a linear C₁- or C₂-alkylene chain, R₅ represents a linear or branched (C₁-C₆)alkyl group, and V, A, E, M and Y are as defined for formula (I),
to yield the compounds of formula (X): 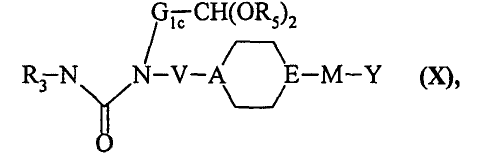 wherein R₃, G_{1c}, V, A, R₅, M and Y are as defined hereinbefore, which compounds of formula (X) are placed in the presence of a strong organic acid
to yield the compounds of formula (I/c) : 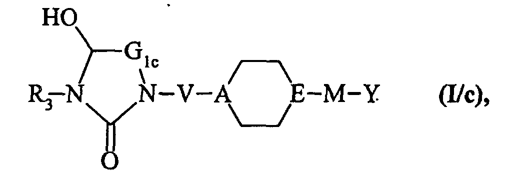 wherein R₃, G_{1c}, V, A, E, M and Y are as defined hereinbefore, which compound of formula (I/c) is hydrogenated according to conventional techniques of organic synthesis
to yield the compounds of formula (I/d), a particular case of the compounds of formula (I) : 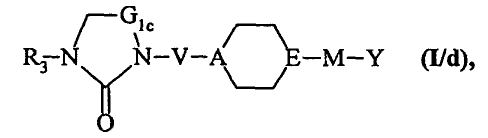 wherein R₃, G_{1c}, V, A, E, M and Y are as defined hereinbefore,
the totality of the compounds of formulae (I/a) and (I/d) constituting the compounds of the invention, which are purified, if necessary, according to conventional purification techniques, are separated, where appropriate, into their isomers according to a conventional separation technique and are converted, if desired, into their hydrates, solvates or addition salts with a pharmaceutically acceptable acid.

7. Pharmaceutical compositions comprising as active ingredient at least one compound of formula (I) according to any one of claims 1 to 5, alone or in combination with one or more inert, non-toxic pharmaceutically acceptable excipients or carriers.

8. Pharmaceutical compositions according to claim 7 comprising at least one active ingredient according to any one of claims 1 to 5 for use as a medicament for the treatment of diseases or pathological conditions in which endothelial dysfunction is known.

9. Pharmaceutical compositions according to claim 7 comprising at least one active ingredient according to any one of claims 1 to 5 for use as a medicament for preventing the development, extension and complications of atherosclerotic lesions, especially in patients exhibiting a vascular risk factor (dyslipidaemia, diabetes, systemic arterial hypertension), for preventing vascular complications after vascular bypass, vascular dilatation, vascular repermeabilisation and heart transplantation or for treating myocardial or peripheral ischaemia, cardiac insufficiency and pulmonary arterial hypertension.
